# EUROPEAN PATENT SPECIFICATION

(11) **EP 3 441 060 B1**
(45) Date of publication and mention of the grant of the patent: **14.06.2023**
(21) Application number: 16898003.5
(22) Date of filing: 08.04.2016
(51) Int. Cl.: A61K 8/89, A61K 8/891, A61K 8/92, A61K 8/34, A61K 8/06, A61Q 19/00, A61K 8/31, A61K 8/37, A61K 8/39, A61K 8/58

(54) **NANO-EMULSION COSMETIC COMPOSITION IN WHICH HIGH CONTENT OILS ARE STABILIZED**
KOSMETISCHE ZUSAMMENSETZUNG IN FORM EINER NANOEMULSION MIT STABILISIERTEN ÖLEN MIT HOHEM GEHALT
COMPOSITION COSMÉTIQUE DE NANO-ÉMULSION DANS LAQUELLE DES HUILES À TENEUR ÉLEVÉE SONT STABILISÉES

(43) Date of publication of application: 13.02.2019
(73) Proprietor: LG Household & Health Care Ltd., Seoul 03184 (KR)
(72) Inventor: BAEK, Woon Ki, Daejeon 34114 (KR); CHOI, Min Sung, Daejeon 34114 (KR); SONG, Young Sook, Daejeon 34114 (KR); PARK, Sun Gyoo, Daejeon 34114 (KR)
(74) Representative: Goddar, Heinz J.
(86) International application number: PCT/KR2016/003678
(87) International publication number: WO 2017/175899

(56) References cited:
- EP-A2- 1 839 644
- WO-A1-2015/152422
- WO-A2-2006/045170
- KR-A- 20110 106 657
- KR-A- 20130 051 705
- KR-A- 20140 115 427
- KR-A- 20160 005 182
- KR-A- 20160 056 843
- US-A1- 2013 011 454
- DATABASE GNPD [Online] MINTEL; 31 January 2018 (2018-01-31), anonymous: "Revitalizing Emulsion", XP055634502, retrieved from www.gnpd.com Database accession no. 5411881

## Description

### [Technical Field]

The present invention relates to a nano-emulsion cosmetic composition in which high content oils are stabilized.

### [Background Art]

An emulsion refers to a liquid-liquid dispersion system in which one or more liquids which are not mixed in one liquid are dispersed, and generally, has a size distribution from several to tens of µm.

A macro emulsion having the above-mentioned particle size is thermodynamically unstable, and ultimately has a property of being separated by various ways such as flocculation, sedimentation, creaming, Ostwald ripening and coalescence.

At this time, when the particle size of a dispersed-phase emulsion is reduced to a nano size, the stability of the emulsion in the kinetic aspect can be greatly improved by Brownian motion, and since a low-viscosity emulsion with a high internal phase is prepared, cosmetics with various textures can be produced. Moreover, due to a small particle size, there is an advantage that an active ingredient can be effectively delivered to the skin.

Generally, although there are slight differences according to literature, liquid-liquid dispersion systems in which dispersed-phase particles have an average diameter of 20 to 500 nm are called nano-emulsions (Flockhart, I. R. etc., Nanoemulsions derived from lanolin show promising drug delivery properties, J. Pharm. Pharmacol., 50 (Supplement) 1998, 141).

There were various attempts to prepare a nano-emulsion with fine particles and low viscosity, and among these attempts, phase inversion temperature emulsification or high-pressure emulsification has been used.

Among these methods, the phase inversion temperature emulsification is an emulsification method using the principle in which the hydrophilic property is reduced by reducing hydrogen bondsbonding between ethylene oxide, which is a hydrophilic group of a non-ionic surfactant, and water when a temperature in a composition consisting of three components such as water, oil and a non-ionic surfactant is increased (Inter. J. Cosmet. Sci. 1994, 16, 84-92). At this time, the three-component composition forms an O/W emulsion at a certain temperature or less, and a W/O emulsion above the certain temperature. This temperature is called a "phase inversion temperature (PIT)."

In addition, although high-pressure emulsification for preparing a nano-emulsion using a high pressure homogenizer was used, there was a limitation in obtaining a translucent preparation by applying high content oil to a low-viscosity preparation such as a toner.

As described above, although various efforts have been made to produce fine and uniform particles, since there are a variety of technical limitations in producing particles with desired viscosity and a desired nano size, and a cosmetic which contains high content oil and has a translucent appearance, these methods were not widely and practically applied in a production aspect.

### [References]

Stefan Schultz and Joachim Ulrich, High pressure homogenizations as a process for emulsion formation, Chem Engineering & Technology, 27, 361-368 (2004)
Wan Goo Cho, Nanoemulsions: Novel Vehicle for Cosmetics, J. Soc. Cosmet. Scientists Korea, 37, 1-21 (2011)
MYu Koroleva, E V Yurtov, Nanoemulsions: The properties, methods of preparation and promising applications, Russian Chemical Reviews, 81, 21-43 (2012).
EP1839644 discloses stable nano-emulsions comprising methylglucose polyglyceryl-3 distearate, lecithin, silicone wax and glycerol.

### [Technical Problem]

Therefore, the inventors developed a low-viscosity oil-in-water (O/W) nano-emulsion cosmetic composition, which has excellent stability due to a very small average emulsion particle size, contains high content oil and has a translucent appearance, by applying high pressure homogenizer Bi-Pass emulsification using polyglyceryl-10 stearate as emulsifier, and thus the present invention was completed.

The object of the present invention is to provide a method of preparing a translucent oil-in-water (O/W) nano-emulsion cosmetic composition according to claim 1.

### [Technical Solution]

The present disclosure provides a method of preparing a translucent oil-in-water (O/W) nano-emulsion cosmetic composition, which includes: mixing water with a polyol or polyol derivative; mixing one or more oils selected from the group consisting of silicone oil, ester-based oil and hydrocarbon-based oil with a polyglyceryl-based emulsifier wherein the polyglyceryl-based emulsifier is polyglyceryl 10-stearate; and mixing the mixture of 1) with the mixture of 2), and then high-pressure emulsifying the resulting mixture, wherein the oil is contained at 5 to 30 wt % based on the total weight of the composition; and wherein the high-pressure emulsification is performed two times or more.

### [Advantageous Effects]

According to the present invention, through a combination method of conventional emulsification and high-pressure emulsification, a translucent nano-emulsion containing high content oil is provided. Accordingly, there is a limitation in solubilization of a large amount of oil, and by using a small amount of oil and large amounts of a polymer and a moisturizer, limitations in a conventional low viscosity cosmetic composition exhibiting various textures can be overcome. Therefore, a cosmetic composition with various textures can be prepared, and can effectively deliver an active ingredient to the skin due to a small emulsion particle size.

### [Description of Drawings]

FIG. 1 illustrates the sizes of nano-emulsion particles according to Example 1.
FIG. 2 illustrates the result of assessing stability with transparency before passing through a high pressure homogenizer, after passing through a high pressure homogenizer one time, and after passing through a high pressure homogenizer second times in preparation of a nano-emulsion according to Example 1.
FIG. 3 illustrates the result of assessing transparency in Comparative Examples 1 to 3.

### [Modes of the Invention]

The present disclosure provides a translucent oil-in-water (O/W) nano-emulsion cosmetic composition, which includes: water; polyglyceryl-10 stearate emulsifier; one or more high content oils selected from the group consisting of silicone oil, ester-based oil and hydrocarbon-based oil; and a polyol

The polyglyceryl-based emulsifier is polyglyceryl-10 stearate.

The polyglyceryl-based emulsifier emulsifier may be included at 1 to 10 wt% with respect to the total weight of the cosmetic composition. Outside the above-mentioned range, the cosmetic composition may have neither a sufficient hardness nor an opaque appearance.

A type of oil used herein is not particularly limited as long as it is conventionally used in preparation of an O/W emulsion composition, and for example, any one or more selected from the group consisting of silicone oil, ester-based oil and hydrocarbon-based oil may be used.

The silicone oil may be one or more selected from the group consisting of dimethicone, cyclomethicone, polydimethylsiloxane, methylphenylpolysiloxane, methylcyclopolysiloxane, octamethylcyclotetrasiloxane, decamethylcyclopentasiloxane, dodecamethylcyclohexasiloxane, tetradecamethylhexasiloxane and octamethyltrisiloxane, but the present invention is not limited thereto.

The ester-based oil may be any one selected from the group consisting of triethylhexanoin, cetyl ethylhexanoate, cetyl octanoate, cetyl isooctanoate, octyldodecyl myristate, pentaerythrityl tetraethyl hexanoate, isopropyl palmitate, isopropyl myristate, caprylic/capric triglyceride, butylene glycol dicaprylate/dicaprate, tocopherol acetate and dicaprylyl carbonate, but the present invention is not limited thereto.

The hydrocarbon-based oil may be one or more selected from the group consisting of paraffin, ceresin and microcrystalline wax, but the present invention is not limited thereto.

The oil is contained at 5 to 30 wt% based on the total weight of the composition. Here, the term "content of oil" refers to the total weight of an oil phase component which does not contain an emulsion stabilizer. When the oil content is less than 5 wt%, the cosmetic composition can be easily obtained without significant differences in appearance and texture from a conventional skin lotion-type cosmetic composition, and when the oil content is more than 30 wt%, due to larger emulsion particles, emulsion stability may be degraded.

In the present invention, as high content oil is used, even when 5 wt% or more of the oil is used, a stabilized translucent emulsion preparation is prepared. According to a conventional technique, stability/translucence was not ensured, and the conventional technique relates to a composition generally containing a low content of oil, such as less than 5%, which is used in nano-emulsification.

The polyol may be glycerin, dihydric alcohol or a mixture thereof. More specifically, the polyol may be one or more selected from the group consisting of butylene glycol, glycerin, propylene glycol, dipropylene glycol, propanediol, hexanediol, and a mixture thereof. Specifically, the polyol included in the composition according to the present invention may be, for example, a mixture of glycerin and 1,2-hexanediol, but the present invention is not limited thereto.

The polyol may be contained at 1 to 30 wt% based on the total weight of the composition. When the content of the polyol is less than 5 wt%, skin moisturizing efficiency may be lacking, and when the content of the polyol is more than 30 wt%, degradation in texture and skin irritation may occur.

In some cases, the composition according to the present invention may further include a pigment, a fragrance, a preservative or a thickening agent as an auxiliary component used in preparation of an O/W nano-emulsion composition. Here, a content of the auxiliary component may be 0 to 20 wt% based on the total weight of the composition.

The water used herein may be contained as the remainder in the composition, based on the total weight of the composition.

In addition, the nano-emulsion according to the present invention may be any one that has low enough viscosity to achieve desired dispersibility to increase the content of an internal phase and effectively deliver an active ingredient, without particular limitation, and for example, the nano-emulsion according to the present invention may have a viscosity of 5000 cps or less, specifically, 3000 cps or less, and more specifically, 2000 cps or less. The emulsion having "low viscosity" includes emulsions which have very low viscosity which cannot be experimentally measured and emulsions which have the above-mentioned range of viscosity. When the viscosity is not experimentally measured, since the viscosity may be 0, the viscosity may be in a range of, for example, 0 to 5000 cps, specifically, 1 to 3000 cps, and more specifically 5 to 2000 cps.

The emulsion is not particularly limited as long as it has a nano size, is enhanced in stability thereof and has low viscosity, and the average size may be, for example, 250 nm or less, and specifically, 200 nm or less. The emulsion may have any size included in the above-mentioned range of the average size, and the average size may be in a range of, for example, 10 to 250 nm, and specifically, 20 to 200 nm. At this time, the particle size distribution may be broadly 10 to 250 nm, and specifically 50 to 150 nm, which is very narrow. Therefore, a nano-emulsion composition which has excellent stability and a light texture may be provided.

The emulsion composition exhibits a translucent appearance. When conventional emulsification or simply high-pressure emulsification is only applied, it is easy to achieve a milky or cloudy appearance, but it is difficult to exhibit a translucent appearance. However, the emulsion composition according to the present invention may have a translucent appearance like a low viscosity skin lotion and also obtain a nutritive texture, which is opposite to that of a skin lotion, using high content oil.

The cosmetic composition according to the present invention has no limitation in its formulation, may be used on skin, a mucous membrane, the scalp or hair, and may be formulated as, for example, a basic cosmetic such as a softening toner, a nourishing toner, a lotion, a cream, a pack, a gel, a patch or a spray (mist); a color cosmetic such as a lipstick, a makeup base or a foundation; a cleansing cosmetic such as a shampoo, a rinse, a body cleanser, a toothpaste or a mouthwash; a hair fixative such as a hair tonic, a hair gel or a hair mousse; or a cosmetic composition for hair such as a hair restorer or a hair colorant. In addition, the cosmetic composition according to the present invention may be widely applied to medicines and quasi-drugs, including a lotion, an ointment, a gel, a cream, a patch or a spray.

The present invention provides a method of preparing a translucent O/W nano-emulsion cosmetic composition by applying emulsification and high-pressure emulsification to the composition.

Specifically, the present invention provides a method of preparing a translucent O/W nano-emulsion cosmetic composition, which includes: mixing water with a polyol or polyol derivative; mixing one or more oils selected from the group consisting of silicone oil, ester-based oil and hydrocarbon-based oil with polyglyceryl-10 stearate emulsifier; and mixing the mixture of 1) with the mixture of 2), and then high-pressure emulsifying the resulting mixture.

In addition, the same contents as those for the above cosmetic composition may be applied to the components described in the preparation method.

The high-pressure emulsification may be performed under a pressure of 500 to 2500 bar. When the pressure is less than 500 bar, the transparency and stability of the appearance cannot be expected, thereby having a problem in formation of a proper nano-emulsion, and when the pressure is more than 2500 bar, it may pose a risk on a high pressure homogenizer, which can be a problem for continuous production.

In addition, the high-pressure emulsification is performed two or more times, so that stabilized particles having an average particle size of 20 to 250 nm may be obtained.

### [Examples]

Hereinafter, the present invention will be described in detail with reference to examples to help in understanding the present invention. The examples of the present invention are provided to more completely explain the present invention to those of ordinary skill in the art.

### Example 1 and Comparative Examples 1 to 3: Preparation of O/W nano-emulsion cosmetic composition

In Example 1 and Comparative Examples 1 to 3, cosmetic compositions were prepared by the following method according to components and contents listed in Table 1 below.

As shown in Table 1 below, first, raw material of the first part were measured, stirred with a disper, and then heated (75 □). Afterward, raw materials of the second part were measured/stirred, heated (75 □), mixed with the raw materials of the first part previously prepared, and then stirred using a Homo-mixer at 3000 rpm for 5 minutes. Raw materials of the third part were input at 50□, stirred under the same conditions (3000 rpm/5 minutes), and then cooled to 30□. The resultant products passed through an M-110P Microfluidizer^{™} high pressure homogenizer two times under 1000 bar, and cooled to 30□.

At this time, instead of a Homo-mixer used in a conventional emulsification system, a high pressure homogenizer was used.

According to the change in the process, physical or physicochemical properties of emulsion particles may be changed.

**[Table 1]**

| Classification (wt%) | Name of raw materials | Example 1 | Comparative Example 1 | Comparative Example 2 | Comparative Example 3 |
|---|---|---|---|---|---|
| First part | Water | to 100 | to 100 | to 100 | to 100 |
| | Glycerin | 8 | 8 | 8 | 8 |
| | 1,2-hexanediol | 2 | 2 | 2 | 2 |
| Second part | Polyglyceryl-10 stearate | 5 | 0 | 0 | 0 |
| | Polysorbate 60 | 0 | 5 | 0 | 0 |
| | Sorbitan stearate/PEG-40 stearate | 0 | 0 | 5 | 0 |
| | Lecithin | 0 | 0 | 0 | 5 |
| | Cetyl ethylhexanoate | 5 | 5 | 5 | 5 |
| | Dimethicone | 5 | 5 | 5 | 5 |
| Third part | Ethanol | 5 | 5 | 5 | 5 |
| | Fragrance | 0.1 | 0.1 | 0.1 | 0.1 |

### Experimental Example 1: Evaluation of stability

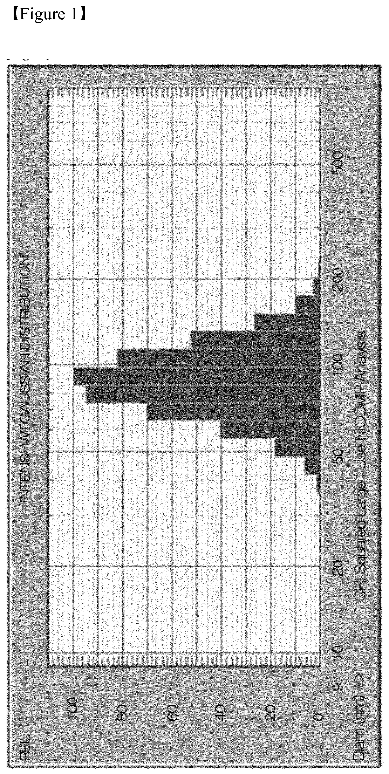

FIG. 1 illustrates the sizes of the nano-emulsion particles of Example 1, which passed through the Homo-mixer and then the high pressure homogenizer two times. This result shows that an average particle size is approximately 100 nm. Conventionally, when the particle size is 200 nm or less, the particles appear translucent and bluish, and thus it can be easily determined by visual observation that a nano emulsion was well prepared. In addition, in the evaluation of formulation stability, it can be determined that, when the translucent appearance was maintained as it was immediately after the preparation, stability is maintained. Here, a particle size was measured using a particle size analyzer (Submicron Particle Sizer NICOMP 380).

At this time, a particle size may vary according to experimental conditions (the contents of components in a composition, the number of times passing through a high pressure homogenizer, etc.). Such measurement of a particle size may be used as another reliable method for judging the stability of a formulation as well as easy judgment by visual observation.

### Experimental Example 2: Confirmation of transparency

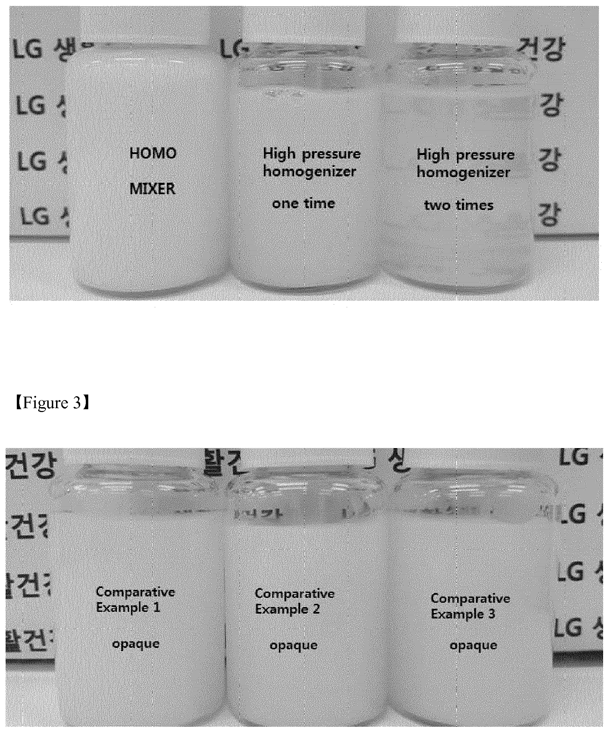

An emulsion was prepared according to Example 1, transparency after storage at 40□ for 30 days was visually observed, and the result is shown in FIG. 2.

In addition, Comparative Examples 1 to 3 passing through the high pressure homogenizer two times showed an opaque appearance unlike the example, and the result is shown in FIG. 3.

### Experimental Example 3: Confirmation of viscosity

The viscosity of a product was measured using a Brookfield DV-E viscometer, and expressed in units of centipoise (cps). The viscosity of a sample measured in this experiment is shown in Table 2 below.

### Experimental Example 4: Confirmation of texture

A portion (approximately 0.5 ml) of each of the emulsion compositions of Example 1 and Comparative Examples 1 to 3 was applied to the back of a hand, and then various aspects of sensory quality, including appearance, spreadability, moistness, oiliness, stickiness, wearability, absorptivity, feel after use, persistency, etc. were assessed, thereby evaluating overall product satisfaction. The texture/characteristics of the example and the comparative examples are shown in Table 2 below.

**[Table 2]**

| Classification | Example 1 | Comparative Examples 1 | Comparative Examples 2 | Comparative Examples 3 |
|---|---|---|---|---|
| Appearance | translucent | opaque | opaque | opaque |
| Viscosity (cps) | 800 cps | 7500 cps | 5500 cps | 6500 cps |
| Texture/characteristics | Changed in texture: | Sticky emulsion | Sticky emulsion | Considerably sticky |
| | starting with rich moistness and finishing with concentrated oiliness | with greater oiliness than moistness | with smooth spreadability | emulsion |
| Stability | Stable Refer to FIG. 2 | Unstable Refer to FIG. 3 | Unstable Refer to FIG. 3 | Unstable Refer to FIG. 3 |

## Claims

1. A method of preparing a translucent oil-in-water (O/W) nano-emulsion cosmetic composition, comprising:
1) mixing water with a polyol;
2) mixing one or more oils selected from the group consisting of silicone oil, ester-based oil and hydrocarbon-based oil with a polyglyceryl-based emulsifier, wherein the polyglyceryl-based emulsifier is polyglyceryl 10-stearate; and
3) mixing the mixture of 1) with the mixture of 2), and then high-pressure emulsifying the resulting mixture,
wherein the oil is contained at 5 to 30 wt% based on the total weight of the composition; and
wherein the high-pressure emulsification is performed two times or more.

2. The method according to claim 1, wherein the high-pressure emulsification is performed under a pressure of 500 to 2500 bar.

3. The method according to claim 1, wherein the emulsifier is contained at 1 to 10 wt% with respect to the total weight of the cosmetic composition.

4. The method according to claim 1, wherein the silicone oil is one or more selected from the group consisting of dimethicone, cyclomethicone, polydimethylsiloxane, methylphenylpolysiloxane, methylcyclopolysiloxane, octamethylcyclotetrasiloxane, decamethylcyclopentasiloxane, dodecamethylcyclohexasiloxane, tetradecamethylhexasiloxane and octamethyltrisiloxane.

5. The method according to claim 1, wherein the ester-based oil is any one selected from the group consisting of triethylhexanoin, cetyl ethylhexanoate, cetyl octanoate, cetyl isooctanoate, octyldodecyl myristate, pentaerythrityl tetraethyl hexanoate, isopropyl palmitate, isopropyl myristate, caprylic/capric triglyceride, butylene glycol dicaprylate/dicaprate, tocopherol acetate and dicaprylyl carbonate.

6. The method according to claim 1, wherein the hydrocarbon-based oil is one or more selected from the group consisting of paraffin, ceresin and microcrystalline wax.

7. The method according to claim 1, wherein the polyol is selected from the group consisting of glycerin, dihydric alcohol and a mixture thereof.

8. The method according to claim 1, wherein the polyol is contained at 1 to 30 wt% based on the total weight of the composition.

## Patentansprüche

1. Verfahren zur Herstellung einer transluzenten Öl-in-Wasser (O/W)-Nanoemulsionskosmetikzusammensetzung, umfassend:
1) Mischen von Wasser mit einem Polyol;
2) Mischen eines oder mehrerer Öle, ausgewählt aus der Gruppe bestehend aus Silikonöl, Öl auf Basis von Ester und Öl auf Basis von Kohlenwasserstoff, mit einem Emulgator auf Basis von Polyglyceryl, wobei der Emulgator auf Basis von Polyglyceryl Polyglyceryl-10-stearat ist; und
3) Mischen der Mischung von 1) mit der Mischung von 2) und dann Emulgieren der resultierenden Mischung unter hohem Druck,
wobei das Öl zu 5 bis 30 Gew.-%, bezogen auf das Gesamtgewicht der Zusammensetzung, enthalten ist; und
wobei die Hochdruckemulgierung zweimal oder mehr durchgeführt wird.

2. Verfahren nach Anspruch 1, bei dem die Hochdruckemulgierung unter einem Druck von 500 bis 2500 bar durchgeführt wird.

3. Verfahren nach Anspruch 1, bei dem der Emulgator zu 1 bis 10 Gew.-%, bezogen auf das Gesamtgewicht der Kosmetikzusammensetzung, enthalten ist.

4. Verfahren nach Anspruch 1, bei dem das Silikonöl eines oder mehrere ist, ausgewählt aus der Gruppe bestehend aus Dimethicon, Cyclomethicon, Polydimethylsiloxan, Methylphenylpolysiloxan, Methylcyclopolysiloxan, Octamethylcyclotetrasiloxan, Decamethylcyclopentasiloxan, Dodecamethylcyclohexasiloxan, Tetradecamethylhexasiloxan und Octamethyltrisiloxan.

5. Verfahren nach Anspruch 1, bei dem das Öl auf Basis von Ester eines ist, ausgewählt aus der Gruppe bestehend aus Triethylhexanoin, Cetylhexanoat, Cetyloctanoat, Cetylisooctanoat, Octyldodecylmyristat, Pentaerythrityltetraethylhexanoat, Isopropylpalmitat, Isopropylmyristat, Capryl-/Caprintriglycerid, Butylenglycoldicaprylat/-dicaprat, Tocopherolacetat und Dicaprylylcarbonat.

6. Verfahren nach Anspruch 1, bei dem das Öl auf Basis von Kohlenwasserstoff eines oder mehrere ist, ausgewählt aus der Gruppe bestehend aus Paraffin, Ceresin und mikrokristallinem Wachs.

7. Verfahren nach Anspruch 1, bei dem das Polyol ausgewählt ist aus der Gruppe bestehend aus Glycerin, zweiwertigem Alkohol und einer Mischung davon.

8. Verfahren nach Anspruch 1, bei dem das Polyol zu 1 bis 30 Gew.-%, bezogen auf das Gesamtgewicht der Zusammensetzung, enthalten ist.

## Revendications

1. Procédé de préparation d'une composition cosmétique de nanoémulsion huile-dans-l'eau (O/W) translucide, consistant à :
1) mélanger de l'eau avec un polyol ;
2) mélanger une ou plusieurs huiles sélectionnées dans le groupe constitué d'huile de silicone, huile à base d'ester et huile à base d'hydrocarbure avec un émulsifiant à base de polyglycéryle, dans lequel l'émulsifiant à base de polyglycéryle est un stéarate de polyglycéryle 10 ; et
3) mélanger le mélange de 1) avec le mélange de 2), puis émulsifier à haute pression le mélange résultant,
dans lequel l'huile est contenue à raison de 5 à 30 % en poids sur la base du poids total de la composition ; et
dans lequel l'émulsification à haute pression est effectuée deux fois ou plus.

2. Procédé selon la revendication 1, dans lequel l'émulsification à haute pression est effectuée sous une pression de 500 à 2500 bar.

3. Procédé selon la revendication 1, dans lequel l'émulsifiant est contenu à raison de 1 à 10 % en poids pour le poids total de la composition cosmétique.

4. Procédé selon la revendication 1, dans lequel l'huile de silicone est une ou plusieurs huiles sélectionnées dans le groupe constitué de diméthicone, cyclométhicone, polydiméthylsiloxane, méthylphénylpolysiloxane, méthylcyclopolysiloxane, octaméthylcyclotetrasiloxane, décaméthylcyclopentasiloxane, dodecaméthylcyclohexasiloxane, tétradécaméthylhexasiloxane et octaméthyltrisiloxane.

5. Procédé selon la revendication 1, dans lequel l'huile à base d'ester est sélectionnée dans le groupe constitué de triéthylhexanoin, éthylhexanoate de cétyle, octanoate de cétyle, isooctanoate de cétyle, myristate d'octyldodécyle, hexanoate de pentaérythrityle tétraéthyle, palmitate d'isopropyle, myristate d'isopropyle, triglycéride caprylique/caprique, dicaprylate/dicaprate de butylène glycol, acétate de tocophérol et carbonate de dicaprylyle.

6. Procédé selon la revendication 1, dans lequel l'huile à base d'hydrocarbure est une ou plusieurs huiles sélectionnées dans le groupe constitué de paraffine, cérésine et cire microcristalline.

7. Procédé selon la revendication 1, dans lequel le polyol est sélectionné dans le groupe constitué de glycérine, alcool dihydrique et un mélange de ces derniers.

8. Procédé selon la revendication 1, dans lequel le polyol est contenu à raison de 1 à 30 % en poids sur la base du poids total de la composition.
